# EUROPÄISCHE PATENTSCHRIFT

(11) **EP 2 150 293 B1**
(45) Veröffentlichungstag und Bekanntmachung des Hinweises auf die Patenterteilung: **25.02.2015**
(21) Anmeldenummer: 08758494.2
(22) Anmeldetag: 13.05.2008
(51) Int. Cl.: A61M 1/36, A61M 5/168, A61M 5/145

(54) **VERFAHREN UND VORRICHTUNG ZUR ÜBERPRÜFUNG DER KORREKTEN ANKOPPLUNG EINER ZUGABEVORRICHTUNG AN EIN THERAPIEGERÄT**
METHOD AND DEVICE FOR CHECKING THE CORRECT COUPLING OF A SUPPLY DEVICE TO A THERAPY DEVICE
PROCÉDÉ ET DISPOSITIF POUR VÉRIFIER LE COUPLAGE CORRECT D'UN DISPOSITIF D'ALIMENTATION À UN APPAREIL THÉRAPEUTIQUE

(30) Priorität: 25.05.2007 DE 102007024463
(43) Veröffentlichungstag der Anmeldung: 10.02.2010
(73) Patentinhaber: Fresenius Medical Care Deutschland GmbH, 61352 Bad Homburg (DE)
(72) Erfinder: NÜRNBERGER, Thomas, 97705 Burkardroth (DE); BLASEK, Marco, 97688 Bad Kissingen (DE); KLÖFFEL, Peter, 97720 Nüdlingen (DE)
(74) Vertreter: Laufhütte, Dieter
(86) Internationale Anmeldenummer: PCT/EP2008/003835
(87) Internationale Veröffentlichungsnummer: WO 2008/145256

(56) Entgegenhaltungen:
- EP-A- 1 348 458
- US-A- 4 846 792
- US-A- 6 090 048
- US-A1- 2005 065 459

## Beschreibung

Die Erfindung betrifft ein Verfahren zur Überprüfung der korrekten Ankopplung einer Zugabevorrichtung an ein Therapiegerät, das einen extrakorporalen Kreislauf aufweist, mit dem die Zugabevorrichtung derart in Verbindung steht, dass mittels der Zugabevorrichtung während des Betriebs des Therapiegerätes ein Mittel in den extrakorporalen Kreislauf einführbar ist, wobei die Zugabevorrichtung vorzugsweise auf der arteriellen Seite eines extrakorporalen Blutkreislaufs vor oder nach der Blutpumpe angekoppelt ist.

Aus der DE 38 37 298 C1 ist ein extrakorporaler Blutkreislauf mit einer Heparinspritze bekannt, die in den Unterdruckbereich auf der Saugseite der in der arteriellen Leitung angeordneten Blutpumpe infundiert, wobei sich an der arteriellen Blutleitung stromaufwärts von der Infusionsstelle eine arterielle Klemme bzw. ein arterielles Ventil und stromabwärts vor der Blutpumpe der Infusionsstelle eine Druckmessstelle befindet. Hier wird im einzelnen beschrieben, wie Heparin während der Blutbehandlung dosiert zugeführt werden kann.

Aus der U.S. 6.558.347 B1 ist eine peristaltische Infusionspumpe zur Infusion eines Medikamentes in einen Patienten bekannt. Hier wird eine Vorrichtung und ein Verfahren zur Überprüfung der korrekten Arbeitsweise einer Fingerpumpe bzw. der korrekten Positionierung des Schlauchs in der Fingerpumpe beschrieben. Dabei wird durch Schließen eines Ventils auf der Druckseite der Pumpe ein abgeschlossenes Schlauchvolumen erzeugt, das durch gezieltes Anlaufen der Pumpe gegen das geschlossene Ventil unter Überdruck gesetzt wird. Der Druck im betreffenden Schlauchabschnitt wird gemessen.

Aus der US 2005/0065459 A1 ist die Überwachung der Zufuhr von Substitutionsflüssigkeit mittels Druckmessungen bekannt. Bei einer an ein Therapiegerät angekoppelten Zugabevorrichtung kommt es entscheidend darauf an, dass die Ankopplung fehlerfrei ist. Insbesondere beim Ankoppeln auf der Saugseite einer im extrakorporalen Kreislauf angeordneten Pumpe kann es zu einer Reihe von Risiken und Fehlern kommen. Folgende mögliche Fehler können hierbei vorkommen:
- Die Zugabevorrichtung arbeitet nicht einwandfrei, wodurch das zuzugebende Mittel fehlerhaft angesaugt wird (Problem des ungewollten Bolus);
- bei einer nicht dicht angeschlossenen Leitung entstehen beim Ansaugen Mikroblasen;
- beim Einsatz von Fremdfabrikaten der Zugabevorrichtung, z. B. von Heparinspritzen von Fremdherstellern, ist ein notwendiger Spielausgleich nicht geschaffen, so dass die Zugabevorrichtung nicht einwandfrei arbeitet.

Aufgabe der vorliegenden Erfindung ist es daher, ein Verfahren und eine Vorrichtung zur Überprüfung der korrekten Ankopplung einer Zugabevorrichtung an ein Therapiegerät zu schaffen.

Erfindungsgemäß wird diese Aufgabe zunächst durch ein Verfahren gemäß Anspruch 1 gelöst. Hier wird bei einem gattungsgemäßen Verfahren mit den Merkmalen des Oberbegriffs des Anspruchs 1 ein Abschnitt des extrakorporalen Kreislaufs in Verbindung mit einem Abschnitt der Zugabevorrichtung gegenüber den anderen Teilen des extrakorporalen Kreislaufs abgeschlossen. In diesem abgeschlossenen Bereich wird der Druck verändert und gemessen.

Grundsätzlich kann im Rahmen der Erfindung ein Abschnitt im Unterdruckbereich auf der Saugseite der Blutpumpe zusammen mit einem Abschnitt der Zugabevorrichtung gegenüber dem anderen Teil des extrakorporalen Kreislaufs abgeschlossen sein. Optional kann aber auch ein Abschnitt im Überdruckbereich auf der Druckseite der Blutpumpe zusammen mit einem Abschnitt der Zugabevorrichtung gegenüber den anderen Teilen des extrakorporalen Kreislaufs abgeschlossen sein.

Vorteilhafte Ausgestaltungen der Erfindung ergeben sich aus den sich an den Hauptanspruch anschließenden Unteransprüchen.

Demnach kann vorzugsweise eine vorbestimmte Abfolge von Druckzuständen im Unter- und Überdruckbereich aufgeprägt und gemessen werden und der gemessene Druckverlauf kann mit einem Sollwertverlauf verglichen werden.

Vorteilhaft handelt es sich bei der Zugabevorrichtung um eine Spritzenpumpe mit einer darin eingesetzten Spritze.

Bei der Pumpe im extrakorporalen Kreislauf handelt es sich vorteilhaft um eine okkludierende Pumpe, insbesondere eine peristaltische Pumpe.

Die Pumpe kann vorteilhaft eine Rollenpumpe oder eine Fingerpumpe sein.

Bei dem aus der Zugabevorrichtung, d. h. vorteilhaft aus der Spritzenpumpe, zugeführten Mittel handelt es sich um ein Antikoagulationsmittel und vorteilhaft um Heparin. Es kann sich bei dem zugeführten Mittel aber auch um ein anderes Medikament, z. B. um ein Eisenpräparat zur medizinischen Zusatzbehandlung, handeln.

Der Druck kann über einen mit dem extrakorporalen Kreislauf in Verbindung stehenden Drucksensor gemessen werden.

Vorteilhaft werden die Schlauchabschnitte der arteriellen Leitung eines Blutschlauchsatzes für ein extrakorporales Blutbehandlungsverfahren den unterschiedlichen Druckzuständen unterworfen.

Die Druckänderungen können durch Vorgabe der Translationsbewegung des Kolbens einer Spritzenpumpe und/oder eines Drehwinkels einer peristaltischen Pumpe erfolgen, wobei die durch die Translation und/oder den Drehwinkel vorgegebene Druckänderungen mit dem Sollverlauf oder Sollwert verglichen wird. Andererseits können vorteilhaft auch die Sollwerte oder Sollwertverlaufe für die Drücke vorgegeben werden und die dafür erforderlichen Translationsbewegungen des Kolbens einer Spritzenpumpe und/oder Drehwinkel einer peristaltischen Pumpe können gemessen werden und mit den vorgegebenen Translationsbewegungs- und/oder Drehwinkelsollwerten oder -sollwertverläufen verglichen werden.

Es kann auch das Drehmoment des Motors zum Antrieb der Spritzenpumpe und/oder der peristaltischen Pumpe über den Motorstrom erfaßt und als Sollwert oder Sollwertverlauf vorgegeben werden.

Weiterhin können die Verfahrensabläufe mittels einer Steuervorrichtung gesteuert werden. Hierdurch ist eine Automatisierung der Druckverlaufsüberprüfung möglich. Anstelle dieser Automatisierung ist aber auch eine manuelle Überprüfung im Rahmen der Erfindung möglich. Die vorteilhaft vorgesehene Steuervorrichtung kann in der Steuerung einer Blutbehandlungsmaschine integriert sein. So kann in besonders vorteilhafter Weise die die Verfahrensabläufe steuernde Steuervorrichtung in einer Blutbehandlungsmaschine nachgerüstet werden.

Die Aufgabe wird auch durch ein Therapiegerät mit den Merkmalen des Anspruchs 12 gelöst. Demnach weist ein Therapiegerät, vorzugsweise eine Dialysemaschine die zur Aufnahme eines extrakorporalen Kreislaufs mit einer Zugabevorrichtung für Konzentrate mit einem Verdünnungsmittel geeignet ist, eine Steuervorrichtung auf, bei der die Steuereinheit derart ausgeführt ist, dass sie automatisiert die korrekte Ankopplung der Zugabevorrichtung mittels eines Verfahrens nach einem der Ansprüche 1 bis 11 überprüft.

Bei der Zugabevorrichtung kann es sich vorteilhaft um eine Spritzenpumpe mit einer darin eingesetzten Spritze handeln.

Die Spritzenpumpe kann über eine Schlauchleitung an eine Schlauchleitung des Therapiegerätes im Unterdruckbereich auf der Saugseite einer Pumpe oder aber auch auf der Druckseite einer Pumpe angeschlossen sein.

Die Spritze kann mit einem Konzentrat gefüllt sein, beispielsweise mit einem Antikoagulationsmittel oder ganz besonders vorteilhaft mit Heparin.

Bei dem Schlauchabschnitt handelt es sich um einen Abschnitt der arteriellen Leitung des Blutschlauchsatzes für ein extrakorporales Blutbehandlungsverfahren.

Ganz besonders vorteilhaft kann das Therapiegerät mit den vorgenannten Merkmalen in der Dialyse, Hämodialyse, Hämofiltration, Hämodiafiltration, zur adsorptiven Blutreinigung, in Transfusionsverfahren oder in Autotransfusionsverfahren verwendet werden.

Weitere Merkmale, Einzelheiten und Vorteile der Erfindung werden anhand eines in der Zeichnung dargestellten Ausführungsbeispiels näher erläutert. Es zeigen:
- Figur 1:: eine schematische Darstellung eines Teils eines Therapiegerätes, in dem die Überprüfung der korrekten Ankopplung einer Zugabevorrichtung überprüft werden kann,
- Figur 2:: einen extrakorporalen Kreislauf in einer Ausführungsvariante und
- Figuren 3 - 6:: Druckverlaufsdiagramme für unterschiedliche Testprozedurergebnisse.

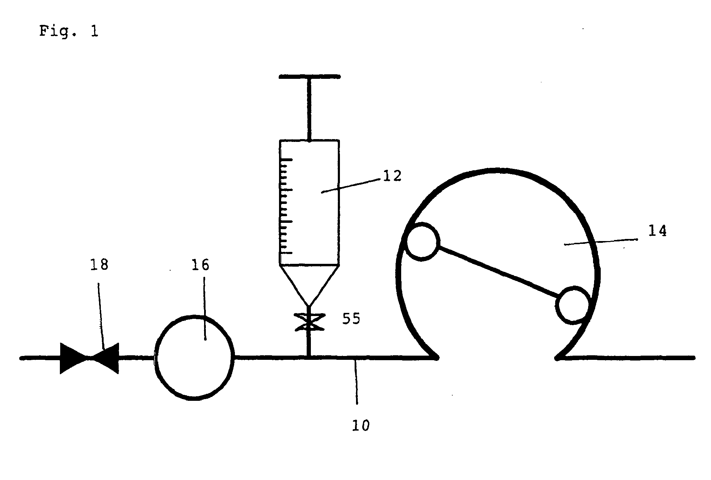

In Figur 1 ist ein Abschnitt der arteriellen Leitung 10 des Blutschlauchsatzes für ein extrakorporales Blutbehandlungsverfahren schematisch dargestellt, an welches eine Spritze 12 angeschlossen ist. Die Spritze 12 ist im Unterdruckbereich einer peristaltischen Pumpe 14, beispielsweise einer okkludierenden Rollenpumpe 14 angeordnet. In der arteriellen Leitung 10 ist weiterhin eine arterielle Druckmeßeinheit 16 angeschlossen. Über eine arterielle Klemme 18 sowie durch die Okklusion der okkludierenden Rollenpumpe 14 und die Spritze 12 kann der hier in Figur 1 dargestellte Teil der arteriellen Leitung gegenüber der Umgebung abgeschlossen werden. Zur Überprüfung der korrekten Ankopplung einer Spritze 12 in einer hier nicht näher dargestellten Spritzenpumpe sowie der Dichtigkeit des Abschnitts des extrakorporalen Blutkreislaufs 10 im Unterdruckbereich auf der Saugseite der Blutpumpe 14 wird der Schlauchabschnitt 10 durch die arterielle Klemme 18, die Spritze 12 und die okkludierenden Blutpumpe 14 abgeschlossen gehalten, wobei der Druck in dem abgeschlossenen Schlauchabschnitt 10 verändert und gemessen wird. Mit Hilfe der Blutpumpe 14 und der Pumpe 12 wird in dem Schlauchabschnitt 10 eine bestimmte Abfolge von Druckzuständen im Unter- und/oder Überdruckbereich aufgeprägt, wobei der gemessene Druckverlauf mit einem Sollverlauf verglichen wird. Bei der hier dargestellten Spritze kann es sich um eine Heparinspritze 12 handeln, in der ein Konzentrat vorgefüllt ist, das nach der entsprechenden Überprüfung und Bestätigung der Dichtigkeit und des funktionell einwandfreien Verlaufs gezielt am extrakorporalen Blutkreislauf aufgezogen und so verdünnt wird, um so die gewünschte Heparinkonzentration, die dann später zugegeben werden soll, zu erzeugen.

Die zu den Testzwecken aufgebrachte Abfolge von Druckzuständen weist bezogen auf den Ausgangsdruck sowohl Überdrücke als auch Unterdrücke auf. Die Unterdrücke können insbesondere durch Rückwärtslauf der Pumpe 12 und/oder durch Vorwärtslauf der Rollenpumpe 14 erzeugt werden. Analog können Überdrücke durch Vorwärtslauf der Spritze 12 und/oder durch Rückwärtslauf der Rollenpumpe 14 erzeugt werden.

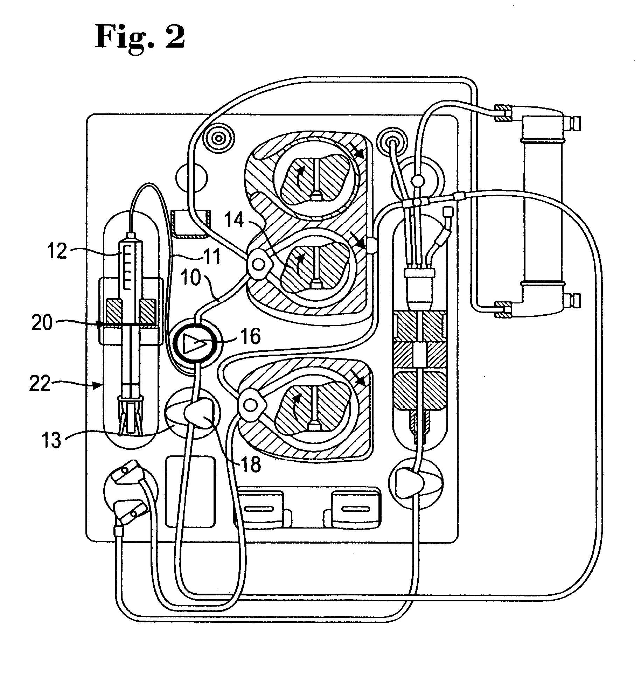

In Figur 2 ist eine konkrete Ausgestaltung des in Figur 1 schematisch dargestellten Bereichs dargestellt. Hier ist eine Spritze 12 im Bereich einer Spritzenpumpe 13 eingelegt, wobei hier die Flügel 20 des Zylinders der Spritze 12 fixiert sind und der Stempel des Spritzenkolbens entsprechend bewegt wird. Über einen Schlauch 11 ist die Spritze 12 mit dem arteriellen Schlauchabschnitt 10 verbunden. Mit 14 ist die Rollenpumpe bezeichnet, über die das arterielle Blut förderbar ist. Der Drucksensor 16 sowie die arterielle Klemme 18 sind in der Vorrichtung gemäß Figur 2 automatisiert. Hier handelt es sich um einen extrakorporalen Blutkreislauf einer Dialysevorrichtung.

Eine Überprüfung der korrekten Ankopplung der Heparinpumpe, d. h. der Spritze 12 erfolgt beispielsweise wie folgt. Die Blutpumpe 14 dreht in die Grundstellung, beispielsweise mit einer Förderrate von 100 ml/min. Anschließend schließt die arterielle Klemme 18. Die Blutpumpe baut gegebenenfalls einen arteriellen Druck auf ≤ 0 mm Hg ab. Dies muss innerhalb eines bestimmten Drehwinkels der Pumpe erreicht werden. Ansonsten ist der Heparinpumpenkonnektionstest gescheitert. Wenn der arterielle Druck abgebaut ist, wird der Blutpumpenstartwinkel gespeichert. Die Blutpumpe erzeugt anschließend einen arteriellen Unterdruck von ≤ -200 mm Hg. Dieser muss vom Startwinkel aus mit maximal 36 ° Drehung der Blutpumpe erreicht werden. Ansonsten ist der Heparinpumpenkonnektionstest gescheitert. Es wird dann 5 Sekunden gewartet, damit sich der Druck stabilisieren kann. Anschließend wird der arterielle Startdruck gespeichert. Die Heparinpumpe, d. h. die Spritze 12 fördert vorwärts bis der arterielle Druck um + 50 mm Hg vom Startdruck gestiegen ist. Dies muss mit einem Fördervolumen der Heparinpumpe, d. h. der Spritze 12, von maximal 2,0 ml erreicht werden. Andernfalls ist der Heparinpumpenkonnektionstest gescheitert.

Die Heparinpumpe, d. h. die Spritze 12, fördert anschließend rückwärts bis der Startdruck wieder erreicht ist. War der Startdruck ≥ -50 mm Hg wird weiter rückwärts gefördert bis der arterielle Druck ≤ -50 mm Hg beträgt. Dies muss mit einem Fördervolumen der Heparinpumpe von maximal 2,0 ml erreicht werden. Andernfalls ist der Konnektionstest gescheitert.

Nach bestandenem oder auch durchgefallenem Heparinpumpenkonnektionstest wird für 2 Sekunden die Blutpumpe angehalten und die Klemmen werden geöffnet, um einen Druckausgleich herzustellen.

Für den Fall, dass der Heparinpumpenkonnektionstest im Vorbereiten bis einschließlichen Zirkulieren nicht durchgeführt wurde bzw. nicht bestanden wurde oder wenn der Anwender die Heparinspritze 12 verspätet aufrüstet, wird beim Übergang in die Dialyse eine Warnung abgegeben, die den Anwender darauf hinweist, dass die Heparinisierung zu prüfen ist.

Grundsätzlich lässt sich der vorgenannte Test auch während der Patientenbehandlung durchführen.

Folgende Fehler können bei dem Drucktest detektiert werden:
- Klemme 55 an der Heparinleitung ist geschlossen;
- Heparinleitung 11 abgeknickt;
- Heparinleitung 11 nicht an Spritze konnektiert;
- Spritzenstempel 22 nicht in Griffstück eingerastet und
- Griffstück nicht an Antrieb angekoppelt (Rastung betätigt).

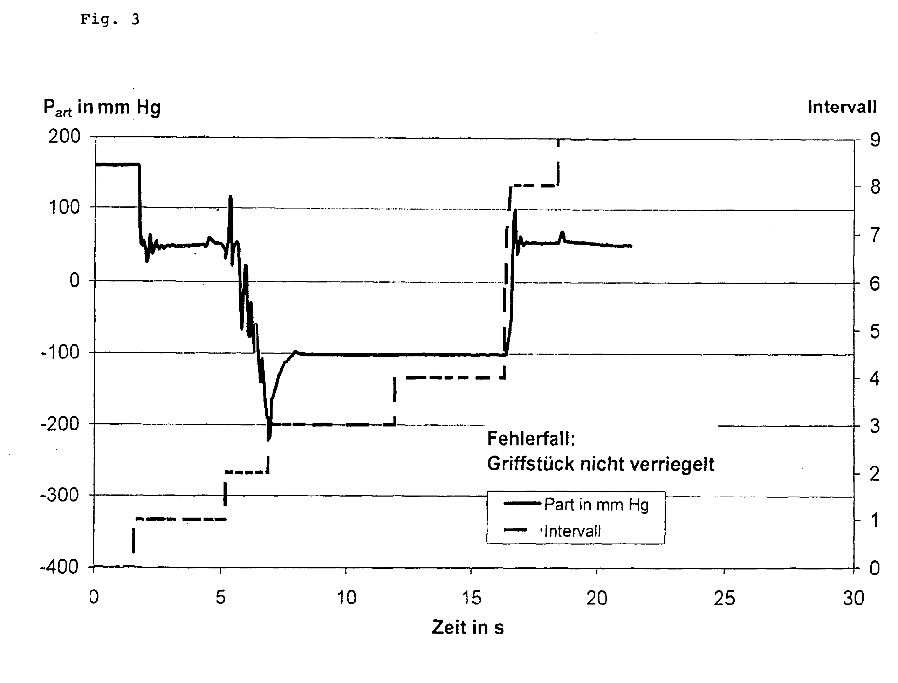

Anhand der Figuren 3 bis 6 werden unterschiedliche Druckverläufe gezeigt, die unterschiedliche Fehlerfälle und auch den Idealfall zeigen.

In Figur 3 kann der geforderte Unterdruck mittels der Blutpumpe nicht innerhalb des Volumenkriteriums aufgebaut werden, da der Drehwinkel der Blutpumpe begrenzt ist. Hier ist der Fehlerfall gezeigt, in dem das Griffstück nicht verriegelt ist oder die Heparinleitung 11 nicht konnektiert ist.

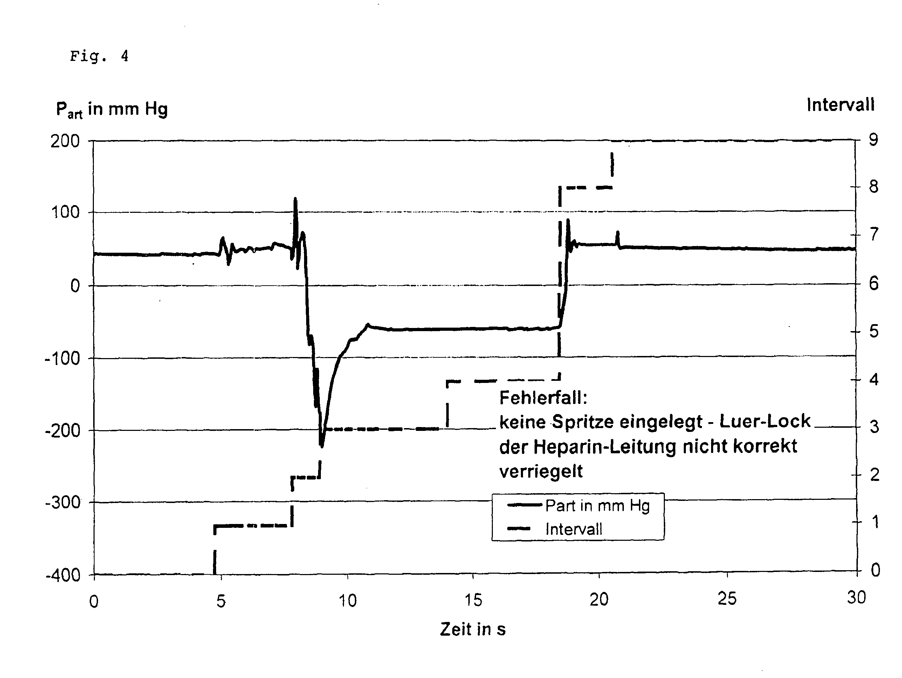

In Figur 4 ist es zwar möglich, den geforderten Unterdruck mittels der Blutpumpe, d. h. der Rollenpumpe, aufzubauen. Der Druckaufbau über die Heparinpumpe, d. h. die Spritze 12, schlägt jedoch fehl, da kein Flüssigkeitsvolumen bewegt wird. Hieraus folgt, dass entweder die Spritze nicht eingelegt ist oder der Luer-Lock der Heparinleitung nicht korrekt verriegelt wurde oder die Heparinleitung mittels einer Klemme 55 abgeklemmt wird.

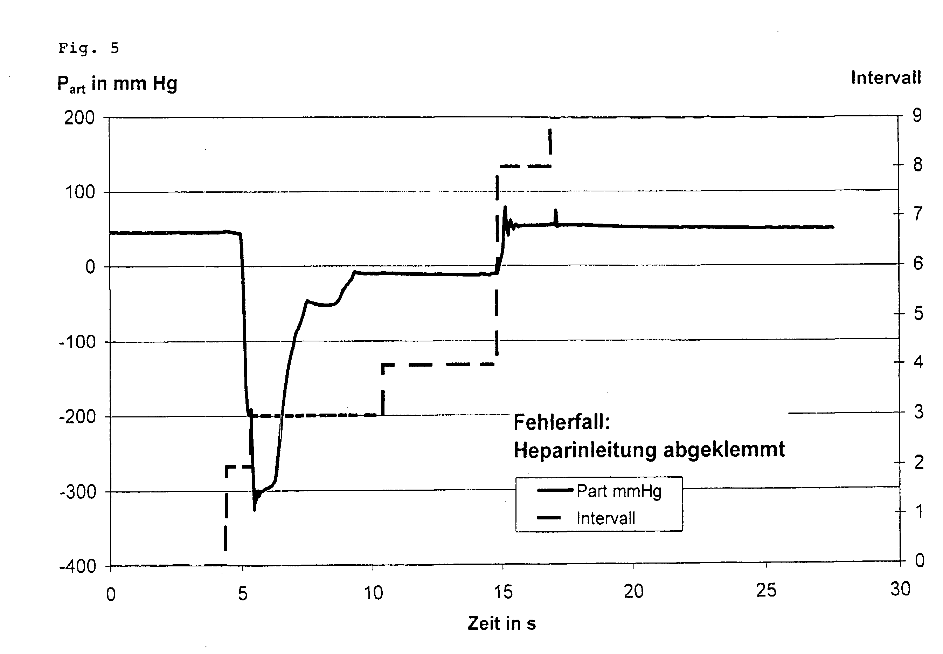

In Figur 5 ergibt das Testergebnis, dass zwar der geforderte Unterdruck mittels der als Rollenpumpe ausgeführten Blutpumpe 14 erreicht ist, dass auch der Druckaufbau über die Spritze 12 funktioniert, dass aber der Unterdruckaufbau mittels der Spritze 12 fehlschlägt, da der Spritzenstempel nicht verriegelt und innerhalb des Volumenkriteriums (Verschiebevolumen der Spritze ist begrenzt) kein größerer Unterdruck aufgebaut werden kann.

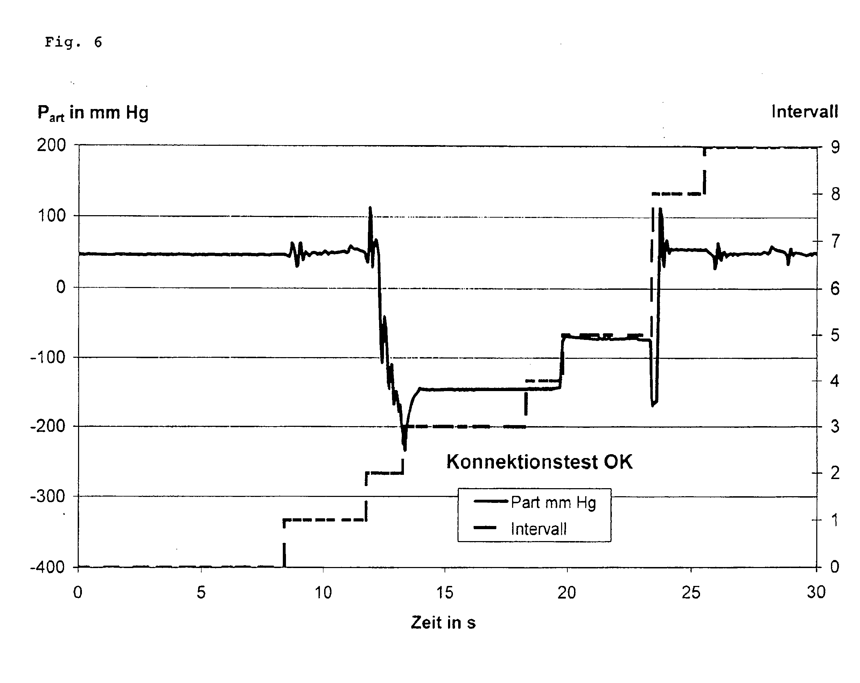

Schließlich wird in Figur 6 der Idealzustand dargestellt, in dem der Konnektionstest erfolgreich abgeschlossen wurde. Hier wird zum einen der geforderte Unterdruck mittels der Blutpumpe erreicht und zum anderen funktioniert auch das Aufprägen des Überdrucks bzw. des Unterdrucks mittels der Spritze 12.

In den Figuren zeigen die gestrichelten stufenförmigen Graphen keine Meßgrößen, sondern unterschiedliche Intervalle der Testprozedur. Die Intervalle betreffen die programmiertechnische Umsetzung der Tests. Es handelt sich bei diesem Beispiel um eine Testprozedur mit drei grundlegenden Schritten, wie zuvor ausgeführt. Es handelt sich hier um folgende Schritte:
- die Blutpumpe baut stromaufwärts einen Unterdruck auf;
- die Heparinpumpe (Spritze) baut einen Überdruck auf;
- die Heparinpumpe (Spritze) baut einen Unterdruck auf.

Dieser Test ist erforderlich, da die Heparinpumpe (Spritze 12) im Unterdruckbereich der Blutpumpe (Rollenpumpe 14) angeordnet ist. Durch diese Anordnung ergeben sich nämlich Nachteile und Risiken, welche durch die oben beschriebenen Tests minimiert werden können.

Die Risiken bzw. Nachteile sind:
- der Stempel der Heparinpumpe ist gegebenenfalls nicht fixiert, dadurch ist ein fehlerhaftes Ansaugen des gesamten Heparins möglich ("ungewollter Bolus);
- die Schlauchklemme in der Heparinleitung ist gegebenenfalls nicht geöffnet, wobei dann unerkannt kein Heparin fließt;
- bei nicht dicht verbundener Heparinleitung entstehen gegebenenfalls Mikroblasen
- ein Spielausgleich ist für Fremdfabrikate der Heparinpumpe nötig

Der Test wurde für minimale Mengen von Heparin optimiert. Durch den Test werden folgende Sachverhalte geprüft:
- Ist die Schlauchklemme in der Heparinleitung geöffnet?
- Ist die Schlauchklemme 55 an der Heparinleitung bei nicht vorhandener Heparinspritze korrekt geschlossen?
- Ist die Heparinspritze korrekt in der Halterung fixiert?
- Können Undichtigkeiten auf der Saugseite der Blutpumpe ausgeschlossen werden?

Die Vorrichtung und das Verfahren, wie sie zuvor dargestellt wurden, betreffen beispielsweise den Blutschlauchsatz und die Dialysemaschine vor der Konnektion des Patienten.

Das vorbeschriebene Verfahren zur Überprüfung der korrekten Ankoppelung kann prinzipiell auch während der Behandlung durchgeführt werden.

## Patentansprüche

1. Verfahren zur Überprüfung der korrekten Ankopplung einer Zugabevorrichtung an ein Therapiegerät, das einen extrakorporalen Kreislauf aufweist, mit dem die Zugabevorrichtung derart in Verbindung steht, dass mittels der Zugabevorrichtung ein Mittel in den extrakorporalen Kreislauf einführbar ist, wobei die Zugabevorrichtung am extrakorporalen Blutkreislauf angekoppelt ist,
**dadurch gekennzeichnet,**
**dass** ein Abschnitt des extrakorporalen Kreislaufs in Verbindung mit einem Abschnitt der Zugabevorrichtung gegenüber den anderen Teilen des extrakorporalen Kreislaufs abgeschlossen wird und dass der Druck in diesem abgeschlossenen Abschnitt verändert und gemessen wird, wobei es sich bei dem Abschnitt um einen Schlauchabschnitt der arteriellen Leitung des Blutschlauchsatzes für ein extrakorporales Blutbehandlungsverfahren handelt.

2. Verfahren nach Anspruch 1, **dadurch gekennzeichnet, dass** eine vorbestimmte Abfolge von Druckzuständen im Unter- und Überduckbereich aufgeprägt und gemessen wird und dass der gemessene Druckverlauf mit einem Sollverlauf verglichen wird.

3. Verfahren nach Anspruch 1 oder 2, **dadurch gekennzeichnet, dass** es sich bei der Zugabevorrichtung um eine Spritzenpumpe mit einer darin eingesetzten Spritze handelt.

4. Verfahren nach einem der vorhergehenden Ansprüche, **dadurch gekennzeichnet, dass** es sich bei der Pumpe um eine okkludierende Pumpe, insbesondere eine peristaltische Pumpe, handelt.

5. Verfahren nach Anspruch 4, **dadurch gekennzeichnet, dass** die Pumpe eine Rollenpumpe oder eine Fingerpumpe ist.

6. Verfahren nach einem der vorhergehenden Ansprüche, **dadurch gekennzeichnet, dass** es sich bei dem in der Spritzenpumpe enthaltenen Mittel um ein Antikoagulationsmittel oder um ein anderes Medikament handelt.

7. Verfahren nach Anspruch 6, **dadurch gekennzeichnet, dass** es sich bei dem Antikoagulationsmittel um Heparin handelt.

8. Verfahren nach einem der vorhergehenden Ansprüche, **dadurch gekennzeichnet, dass** über einen mit dem extrakorporalen Kreislauf in Verbindung stehenden Drucksensor der Druck gemessen wird.

9. Verfahren nach einem der vorhergehenden Ansprüche, **dadurch gekennzeichnet, dass** Schlauchabschnitte der arteriellen Leitung eines Blutschlauchsatzes für ein extrakorporales Blutbehandlungsverfahren nach Abschließen über eine Klemme einerseits und die Spritzenpumpe und die okkludierende Blutpumpe andererseits den unterschiedlichen Druckzuständen unterworfen werden.

10. Verfahren nach einem der vorhergehenden Ansprüche, **dadurch gekennzeichnet, dass** die Druckänderungen durch die Vorgabe der Translationsbewegung des Kolbens einer Spritzenpumpe und/oder eines Drehwinkels einer peristaltischen Pumpe erfolgt und dass die durch die Translation und/oder den Drehwinkel vorgegebene Druckänderung mit dem Sollverlauf oder Sollwert verglichen wird.

11. Verfahren nach einem der vorhergehenden Ansprüche, **dadurch gekennzeichnet, dass** die Sollwerte oder Sollwertverläufe für die Drücke vorgegeben werden und die dafür erforderlichen Translationsbewegungen des Kolbens einer Spritzenpumpe und/oder Drehwinkel einer peristaltischen Pumpe gemessen werden und mit vorgegebenen Translationsbewegungs- und/oder Drehwinkelsollwerten oder -sollwertverläufen verglichen werden.

12. Verfahren nach einem der vorhergehenden Ansprüche, **dadurch gekennzeichnet, dass** das Drehmoment des Motors zum Antrieb der Spritzenpumpe und/oder der peristaltischen Pumpe über den Motorstrom erfaßt und als Sollwert oder Sollwertverlauf vorgegeben wird.

13. Verfahren nach einem der vorhergehenden Ansprüche, **dadurch gekennzeichnet, dass** die Verfahrensabläufe mittels einer Steuervorrichtung gesteuert werden.

14. Verfahren nach einem der vorhergehenden Ansprüche, **dadurch gekennzeichnet, dass** die Steuervorrichtung in der Steuerung einer Blutbehandlungsmaschine integriert ist.

15. Therapiegerät, vorzugsweise Dialysemaschine, geeignet zur Aufnahme eines extrakorporalen Kreislaufes mit einer Zugabevorrichtung für ein Mittel, **dadurch gekennzeichnet, dass** das Therapiegerät eine Steuervorrichtung aufweist, wobei die Steuereinheit derart ausgeführt ist, dass sie automatisiert die korrekte Ankopplung der Zugabevorrichtung mittels eines Verfahrens nach einem der Ansprüche 1 bis 14 überprüft, wobei es sich bei der Schlauchleitung des Therapiegerätes um einen Abschnitt der arteriellen Leitung des Blutschlauchsatzes für ein extrakorporales Blutbehandlungsverfahren handelt.

16. Therapiegerät nach Anspruch 15, **dadurch gekennzeichnet, dass** es sich bei der Zugabevorrichtung um eine Spritzenpumpe mit einer darin eingesetzten Spritze handelt.

17. Therapiegerät nach Anspruch 16, **dadurch gekennzeichnet, dass** die Spritzenpumpe über eine Schlauchleitung an eine Schlauchleitung des Therapiegerätes im Unterdruckbereich auf der Saugseite einer Pumpe angeschlossen ist.

18. Therapiegerät nach Anspruch 16, **dadurch gekennzeichnet, dass** die Spritze mit einem Konzentrat oder einer Lösung gefüllt ist.

19. Therapiegerät nach Anspruch 15, **dadurch gekennzeichnet, dass** es sich bei dem Mittel um ein Antikoagulationsmittel oder um ein anderes Medikament handelt.

20. Therapiegerät nach Anspruch 19, **dadurch gekennzeichnet, dass** es sich bei dem Antikoagulationsmittel um Heparin handelt.

21. Therapiegerät nach einem der Ansprüche 15 bis 20 zur Verwendung in der Dialyse und/oder Hämodialyse, und/oder Hämofiltration, und/oder Hämodiafiltration, und/oder zur adsorptiven Blutreinigung, und/oder in Transfusionsverfahren und/oder in Autotransfusionsverfahren.

## Claims

1. A method for checking the correct coupling of an adding device to a therapeutic appliance, which includes an extracorporeal circuit with which the adding device is connected such that a drug can be introduced into the extracorporeal circuit by means of the adding device, which is coupled to the extracorporeal circuit,
**characterized in**
**that** a portion of the extracorporeal circuit in connection with a portion of the adding device is closed with respect to the other parts of the extracorporeal circuit and that the pressure in this closed portion is varied and measured.

2. The method as claimed in claim 1, **characterized in that** a predetermined sequence of pressure conditions in the region of negative and excess pressure is applied and measured, and that the measured pressure profile is compared with a set-point profile.

3. The method as claimed in claim 1 or 2, **characterized in that** the adding device is a syringe pump with a syringe inserted therein.

4. The method as claimed in any of the preceding claims, **characterized in that** the pump is an occluding pump, in particular a peristaltic pump.

5. The method as claimed in claim 4, **characterized in that** the pump is a roller pump or a finger pump.

6. The method as claimed in any of the preceding claims, **characterized in that** the drug contained in the syringe pump is an anticoagulant or some other medicament.

7. The method as claimed in claim 6, **characterized in that** the anticoagulant is heparin.

8. The method as claimed in any of the preceding claims, **characterized in that** the pressure is measured via a pressure sensor connected with the extracorporeal circuit.

9. The method as claimed in any of the preceding claims, **characterized in that** tube portions of the arterial conduit of a set of blood tubes for a method of extracorporeal blood treatment are subjected to the different pressure conditions after closing via a clamp on the one hand and via the syringe pump and the occluding blood pump on the other hand.

10. The method as claimed in any of the preceding claims, **characterized in that** the changes in pressure are effected by specifying the translational movement of the piston of a syringe pump and/or an angle of rotation of a peristaltic pump and that the change in pressure specified by the translation and/or the angle of rotation is compared with the set-point profile or the set point.

11. The method as claimed in any of the preceding claims, **characterized in that** the set points or set-point profiles for the pressures are specified and the required translational movements of the piston of a syringe pump and/or the angles of rotation of a peristaltic pump are measured and compared with specified set points or set-point profiles of the translational movement and/or angle of rotation.

12. The method as claimed in any of the preceding claims, **characterized in that** the torque of the motor for driving the syringe pump and/or the peristaltic pump is detected via the motor current and specified as a set point or set-point profile.

13. The method as claimed in any of the preceding claims, **characterized in that** the process sequences are controlled by means of a control device.

14. The method as claimed in any of the preceding claims, **characterized in that** the control device is integrated in the control of a blood treatment machine.

15. A therapeutic appliance, preferably a dialysis machine, suitable for accommodating an extracorporeal circuit with an adding device for a drug, **characterized in that** the therapeutic appliance includes a control device, wherein the control unit is designed such that it automatically checks the correct coupling of the adding device by means of a method as claimed in any of claims 1 to 14.

16. The therapeutic appliance as claimed in claim 15, **characterized in that** the adding device is a syringe pump with a syringe inserted therein.

17. The therapeutic appliance as claimed in claim 16, **characterized in that** via a tube conduit the syringe pump is connected to a tube conduit of the therapeutic appliance in the region of negative pressure on the suction side of a pump.

18. The therapeutic appliance as claimed in claim 16, **characterized in that** the syringe is filled with a concentrate or a solution.

19. The therapeutic appliance as claimed in claim 15, **characterized in that** the drug is an anticoagulant or some other medicament.

20. The therapeutic appliance as claimed in claim 19, **characterized in that** the anticoagulant is heparin.

21. The therapeutic appliance as claimed in any of claims 15 to 20 for use in dialysis and/or hemodialysis and/or hemofiltration and/or hemodiafiltration and/or for blood purification by adsorption and/or in transfusion methods and/or in autotransfusion methods.

## Revendications

1. Procédé pour vérifier le couplage correct d'un dispositif d'alimentation à un appareil thérapeutique, qui comporte un circuit extracorporel, auquel le dispositif d'alimentation est relié de telle manière qu'au moyen du dispositif d'alimentation une substance peut être introduite dans le circuit extracorporel, le dispositif d'alimentation étant couplé au circuit sanguin extracorporel,
**caractérisé**
**en ce qu'**une portion du circuit extracorporel en liaison avec une portion du dispositif d'alimentation est fermée par rapport aux autres parties du circuit extracorporel et en ce que la pression dans cette portion fermée est modifiée et mesurée, la portion étant une portion de tube de la ligne artérielle de l'ensemble de tubes sanguins pour un procédé de traitement du sang extracorporel.

2. Procédé selon la revendication 1, **caractérisé en ce qu'**une suite prédéfinie d'états de pression dans le domaine de dépression et le domaine de surpression est imposée et mesurée et **en ce que** la variation de pression mesurée est comparée à une variation de consigne.

3. Procédé selon la revendication 1 ou 2, **caractérisé en ce que** le dispositif d'alimentation est une pompe seringue comprenant une seringue placée dans celle-ci.

4. Procédé selon l'une des revendications précédentes, **caractérisé en ce que** la pompe est une pompe occlusive, en particulier une pompe péristaltique.

5. Procédé selon la revendication 4, **caractérisé en ce que** la pompe est une pompe à galets ou une pompe à doigts.

6. Procédé selon l'une des revendications précédentes, **caractérisé en ce que** la substance contenue dans la pompe seringue est un anticoagulant ou un autre médicament..

7. Procédé selon la revendication 6, **caractérisé en ce que** l'anticoagulant est de l'héparine.

8. Procédé selon l'une des revendications précédentes, **caractérisé en ce que** la pression est mesurée par le biais d'un capteur de pression qui est en liaison avec le circuit extracorporel.

9. Procédé selon l'une des revendications précédentes, **caractérisé en ce que** des portions de tube de la ligne artérielle d'un ensemble de tubes sanguins pour un procédé de traitement sanguin extracorporel sont soumises aux différents états de pression après fermeture d'un côté par une pince et de l'autre côté par la pompe seringue et la pompe à sang occlusive.

10. Procédé selon l'une des revendications précédentes, **caractérisé en ce que** les modifications de pression sont effectuées par la contrainte du mouvement de translation du piston d'une pompe seringue et/ou d'un angle de rotation d'une pompe péristaltique et **en ce que** la modification de pression prédéfinie par la translation et/ou l'angle de rotation est comparée à la variation de consigne ou à la valeur de consigne.

11. Procédé selon l'une des revendications précédentes, **caractérisé en ce que** les valeurs de consigne ou les variations de valeur de consigne pour les pressions sont prédéfinies et les mouvements de translation du piston d'une pompe seringue et/ou angles de rotation d'une pompe péristaltique nécessaires pour cela sont mesurés et sont comparés à des valeurs de consigne ou variations de valeur de consigne de mouvement de translation et/ou d'angle de rotation.

12. Procédé selon l'une des revendications précédentes, **caractérisé en ce que** le couple du moteur destiné à entraîner la pompe seringue et/ou la pompe péristaltique est acquis par le biais du courant de moteur et est prédéfini comme valeur de consigne ou variation de valeur de consigne.

13. Procédé selon l'une des revendications précédentes, **caractérisé en ce que** le déroulement du procédé est commandé au moyen d'un dispositif de commande.

14. Procédé selon l'une des revendications précédentes, **caractérisé en ce que** le dispositif de commande est intégré dans la commande d'une machine de traitement du sang.

15. Appareil thérapeutique, de préférence machine de dialyse, adapté pour recevoir un circuit extracorporel comprenant un dispositif d'alimentation pour une substance, **caractérisé en ce que** l'appareil thérapeutique comporte un dispositif de commande, l'unité de commande étant conçue de telle manière qu'elle vérifie automatiquement le couplage correct du dispositif d'alimentation au moyen d'un procédé selon l'une des revendications 1 à 14, le tuyau flexible de l'appareil thérapeutique étant une portion de la ligne artérielle de l'ensemble de tubes sanguins pour un procédé de traitement du sang extracorporel.

16. Appareil thérapeutique selon la revendication 15, **caractérisé en ce que** le dispositif d'alimentation est une pompe seringue comprenant une seringue placée dans celle-ci.

17. Appareil thérapeutique selon la revendication 16, **caractérisé en ce que** la pompe seringue est reliée via un tuyau flexible à un tuyau flexible de l'appareil thérapeutique dans le domaine de dépression du côté aspiration d'une pompe.

18. Appareil thérapeutique selon la revendication 16, **caractérisé en ce que** la seringue est remplie d'un concentré ou d'une solution.

19. Appareil thérapeutique selon la revendication 15, **caractérisé en ce que** la substance est un anticoagulant ou un autre médicament.

20. Appareil thérapeutique selon la revendication 19, **caractérisé en ce que** l'anticoagulant est de l'héparine.

21. Appareil thérapeutique selon l'une des revendications 15 à 20 destiné à l'utilisation dans la dialyse et/ou l'hémodialyse, et/ou l'hémofiltration, et/ou l'hémodiafiltration, et/ou pour l'épuration du sang par adsorption, et/ou dans des procédés de transfusion et/ou des procédés d'autotransfusion.
